(19) Europäisches Patentamt
European Patent Office
Office européen des brevets

(11) Veröffentlichungsnummer: **0 054 512**
**A2**

(12) **EUROPÄISCHE PATENTANMELDUNG**

(21) Anmeldenummer: 81810484.6

(22) Anmeldetag: 07.12.81

(51) Int. Cl.³: C 07 D 501/00
A 61 K 31/545

(30) Priorität: 12.12.80 CH 9196/80

(43) Veröffentlichungstag der Anmeldung: 23.06.82 Patentblatt 82/25

(84) Benannte Vertragsstaaten: AT BE CH DE FR IT LI LU NL SE

(71) Anmelder: CIBA-GEIGY AG
Patentabteilung Postfach
CH-4002 Basel(CH)

(72) Erfinder: Scartazzini, Riccardo, Dr.
Conrad Ferdinand Meyer-Strasse 38
CH-4059 Basel(CH)

(54) **Cephalosporinester, Verfahren zu ihrer Herstellung und sie enthaltende pharmazeutische Präparate.**

(57) Die Erfindung betrifft neue, unter physiologischen Bedingungen spaltbare 1-Niederalkoxycarbonyloxyniederalkylester der 7β-[2-(2-Amino-4-thiazolyl)-2-(syn)-methoxyimino-acetamido]-3-cephem-4-carbonsaüre, Verfahren zu ihrer Herstellung und pharmazeutische Präparate, die solche Verbindungen enthalten.

EP 0 054 512 A2

CIBA-GEIGY AG 4-13190/+

Basel (Schweiz)

BEZEICHNUNG GEÄNDERT
siehe Titelseite

Neue Ester

Die Erfindung betrifft neue, unter physiologischen Bedingungen spaltbare Ester der 7β-[2-(2-Amino-4-thiazolyl)-2-(syn)-methoxyimino-acetamido]-3-cephem-4-carbonsäure, Verfahren zu ihrer Herstellung und pharmazeutische Präparate, die solche Verbindungen enthalten.

Die 7β-[2-(2-Amino-4-thiazolyl)-2-(syn)-methoxyiminoacetamido]-3-cephem-4-carbonsäure, das Natriumsalz davon (Ceftizoxime), einige weitere Salze und auch Ester, einschliesslich einiger physiologisch spaltbarer Ester, sind aus der deutschen Offenlegungsschrift 28 10 922 bzw. aus der europäischen Patentanmeldung 2 273 bekannt. Diese Verbindungen sind wertvolle, antibiotisch wirksame Substanzen, die insbesondere als antibakterielle Antibiotika verwendet werden können. Während die Salze nur zur parenteralen Applikation geeignet sind, haben die unter physiologischen Bedingungen spaltbaren Ester den Vorteil auch nach oraler Application wirksam zu sein.

Es besteht ein Bedürfnis nach oral verabreichbaren Verbindungen dieses Types, die gegenüber den bekannten oralen Cephalosporinen, z.B. vom Phenylglycintyp, verbesserte Eigenschaften aufweisen. Ueberraschender Weise wurde gefunden, dass mit gewissen Estern die pharmakologischen Eigenschaften wesentlich verbessert werden können.

Die vorliegende Erfindung betrifft die neuen Verbindungen der Formel

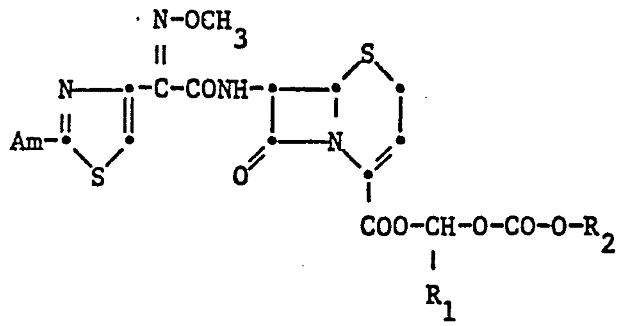

(I),

worin $R_1$ Wasserstoff oder Niederalkyl, $R_2$ Niederalkyl und Am eine gegebenenfalls geschützte Aminogruppe bedeuten, Säureadditionssalze von solchen Verbindungen mit salzbildender Gruppe, Verfahren zu ihre Herstellung und pharmazeutische Präparate, die solche Verbindungen enthalten.

In der vorliegenden Beschreibung der Erfindung bedeutet der Ausdruck "Nieder" in Gruppen wie Niederalkyl, Niederalkylen, Niederalkoxy, Niederalkanoyl und dergleichen, dass die entsprechenden Gruppen, sofern nicht ausdrücklich anders definiert, bis zu 7, bevorzugt bis zu 4 C-Atome enthalten.

Eine Niederalkylgruppe $R_1$ oder $R_2$ ist insbesondere $C_1$-$C_4$-Niederalkyl wie Methyl, Aethyl, Propyl, Isopropyl oder Butyl. Bevorzugt ist $R_1$ Methyl und $R_2$ Aethyl.

In einer geschützten Aminogruppe Am ist die Schutzgruppe eine der in der Penicillin-, Cephalosporin- und Peptidchemie verwendeten.

Eine solche Schutzgruppe ist leicht, das heisst ohne dass unerwünschte Nebenreaktionen stattfinden, beispielsweise solvolytisch, reduktiv oder photolytisch abspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lübke, Academic Press, London, New York, 1965, sowie in "Methoden der organischen Chemie", Houben-Weyl, 4. Auflage, Bd. 16/1, Georg Thieme Verlag, Stuttgart, 1974.

Eine geschützte Aminogruppe Am kann z.B. in Form einer leicht spaltbaren Acylamino-, Arylmethylamino-, verätherten Mercaptoamino-, 2-Acyl-niederalk-1-en-1-yl-amino-, Silyl- oder Stannylaminogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogen-niederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung der Niederalkylreste verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.-Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitrobenzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Diphenylniederalkylphosphoryl, z.B. Dibenzylphosphoryl oder Di-4-nitrobenzylphosphoryl, gegebenenfalls substituiertes Phenyloxy-phenyl-phosphonyl, z.B. Phenyloxy-phenyl-phosphonyl, Diniederalkylphosphinyl, z.B. Diäthylphosphinyl, oder gegebenenfalls substituiertes Diphenylphosphinyl, z.B. Diphenylphosphinyl.

In einer Arylmethylaminogruppe, die Mono-, Di- oder insbesondere Triarylmethylamino darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Eine Silyl- oder Stannylaminogruppe ist in erster Linie eine organische Silyl- bzw. Stannylaminogruppe, worin das Silicium- bzw. Zinnatom vorzugweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Line Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlor-silyl, oder entsprechend substituiertes Stannyl, z.B. Tri-n-butylstannyl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgrppen sind Acylreste von Kohlensäurehalbestern insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichloräthoxycarbonyl, ferner Trityl oder Formyl.

Verbindungen der Formel I mit einer basischen Aminogruppe Am können Säureadditionssalze, z.B. mit starken anorganischen Säuren, wie Chlorwasserstoff- oder Bromwasserstoffsäure, Schwefelsäure oder Phosphorsäure, oder mit geeigneten organischen Carbon- oder Sulfonsäuren, z.B. Trifluoressigsäure oder Methansulfonsäure bilden.

Die Methoxyiminomethylengruppe liegt bevorzugt in der syn-Form (Z-Form) vor.

Die Verbindungen der Formel I, worin Am eine freie Aminogruppe ist und ihre pharmazeutisch verwendbaren, nichttoxischen Salze sind wertvolle, antibiotisch wirksame Substanzen, die insbesondere als antibakterielle Antibiotika verwendet werden können. Beispielsweise hat die Verbindung der Formel I, worin Am eine freie Aminogruppe, $R_1$ Methyl und $R_2$ Aethyl bedeutet, in vivo, bei oraler Applikation an der Maus, bei systemischen gramnegativen Infektionen, z.B. durch Escherichia coli, Proteus spp. und anderen, einen $ED_{50}$-Wert von 0.3 bis 150 mg/kg.

Die neuen Verbindungen können daher als antibakterielle orale Antibiotika, z.B. in Form von oral applizierbaren pharmazeutischen Präparaten, wie Kapseln, Tabletten, Sirups oder dergleichen, zur Bekämpfung von entsprechenden Infektionen Verwendung finden.

Verbindungen der Formel (I), worin Am eine geschützte Aminogruppe ist, werden als Ausgangsmaterialien zur Herstellung von antibiotisch wirksamen Verbindungen der Formel I verwendet.

Die physiologisch spaltbaren Ester der vorliegenden Erfindung zeichnen sich durch eine überraschend gute Resorption nach oraler Verabreichung aus, wie durch die hohe renale Ausscheidung nachgewiesen wird.

Die vorliegende Erfindung betrifft in erster Linie diejenigen Verbindungen der Formel I, worin Am eine freie Aminogruppe, $R_1$ Wasserstoff oder insbesondere Methyl und $R_2$ Aethyl bedeutet, und ihre pharmazeutisch annehmbaren Salze, sowie die entsprechenden Verbindungen mit geschützten funktionellen Gruppen.

Besonders hervorzuheben ist die Verbindung der Formel (I), worin Am die freie Aminogruppe, $R_1$ Methyl und $R_2$ Aethyl bedeuten, und ihre pharmazeutisch annehmbaren Salze.

Die Erfindung betrifft insbesondere die in den Beispielen beschriebenen Verbindungen der Formel I und deren pharmazeutisch annehmbaren Salze.

Die Verbindungen der vorliegenden Erfindung werden nach an sich bekannten Verfahren hergestellt.

So werden Verbindungen der Formel I hergestellt, indem man
a) die 7β-Aminogruppe in einer Verbindung der Formel

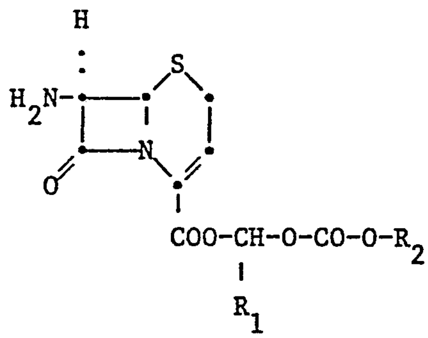

(II),

worin die Aminogruppe gegebenenfalls durch eine die Acylierung erlaubende Gruppe substituiert ist und worin $R_1$ und $R_2$ die unter Form gegebenen Bedeutungen haben, durch Behandeln mit einem den Acylrest einer Carbonsäure der Formel

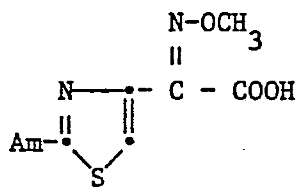

(III)

einführenden Acylierungsmittel, worin Am die unter Formel I angegebenen Bedeutungen hat, acyliert, oder

b) eine Verbindung der Formel

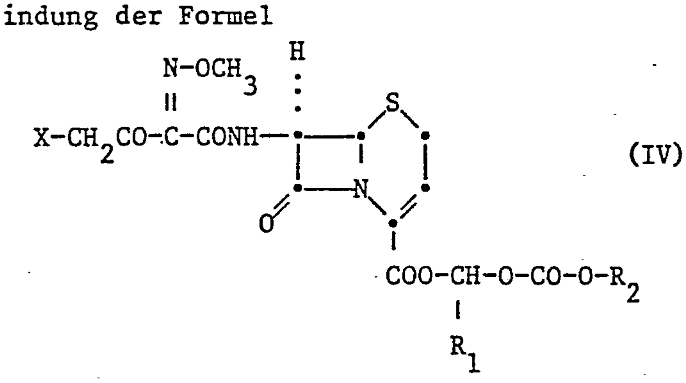

(IV),

worin X Halogen bedeutet, $R_1$ und $R_2$ die unter Formel I genannten Bedeutungen haben, mit einem Thioharnstoff der Formel $Am-CS-NH_2$ ode einem Salz davon kondensiert, oder

c) aus einer Verbindung der Formel

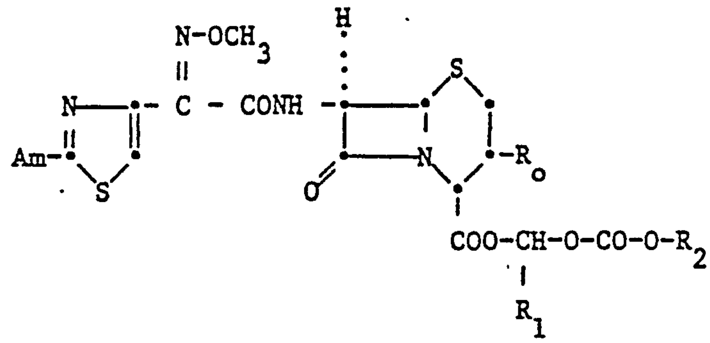

(V),

worin Am, $R_1$ und $R_2$ die unter Formel I angegebenen Bedeutungen haben und $R_o$ Hydroxy, verestertes Hydroxy oder gegebenenfalls substituiertes Amino ist, eine Gruppe $H-R_o$ abspaltet, oder

d) zur Herstellung einer Verbindung der Formel I, worin Am, $R_1$ und $R_2$ die unter Formel I gegebenen Bedeutungen haben, in einer Verbindung der Formel

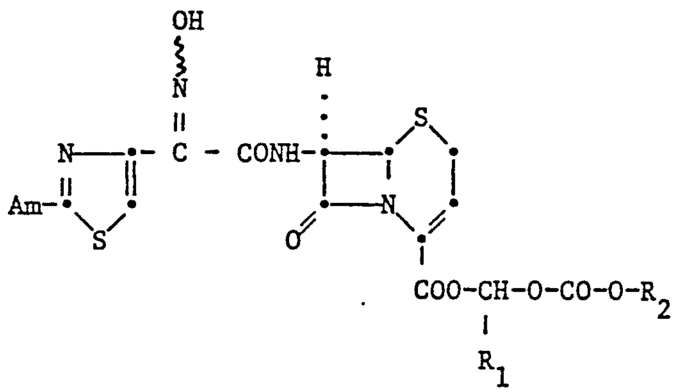

(VI),

worin Am, $R_1$ und $R_2$ die unter Formel I gegebenen Bedeutungen haben, die Hydroxyiminogruppe methyliert, oder

e) zur Herstellung einer Verbindung der Formel I, worin Am, $R_1$ und $R_2$ die unter Formel I genannten Bedeutungen haben, in einer Verbindung der Formel

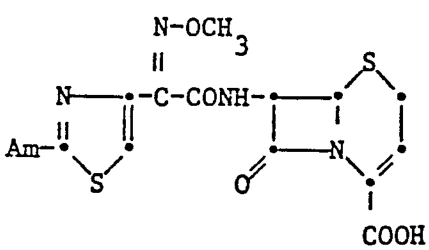

(VII),

worin Am die unter Formel I gegebene Bedeutung hat, die gegebenenfa in Salzform vorliegende Carboxylgruppe in 4-Stellung des Cephemringe durch Behandlung mit einem Veresterungsmittel der Formel

$$\begin{array}{l} X\text{-}CH\text{-}O\text{-}CO\text{-}O\text{-}R_2 \\ \quad\;| \\ \quad\;R_1 \end{array} \qquad (VIII),$$

worin X eine reaktionsfähige, veresterte Hydroxygruppe ist und $R_1$ u $R_2$ die unter Formel I angegebenen Bedeutungen haben, in eine veresterte Carboxylgruppe überführt, und/oder

f) zur Herstellung einer Verbindung der Formel I, worin Am eine frei Aminogruppe ist und $R_1$ und $R_2$ die unter Formel I gegebenen Bedeutungen haben, aus einer Verbindung der Formel I, worin Am eine geschützte Aminogruppe bedeutet, und $R_1$ und $R_2$ die unter Formel I gegebenen Bedeutungen haben, die Aminoschutzgruppe abspaltet und du Wasserstoff ersetzt, und, wenn erwünscht, eine erhaltene Verbindung worin Am eine freie Aminogruppe ist in ein Salz oder ein erhaltenes Salz in eine freie Verbindung oder in ein anderes Salz überführt.

Verfahren a): Gegebenenfalls vorhandene, die Aminogruppe substituierende und deren Acylierung erlaubende Reste in einem Ausgangsmaterial der Formel II sind beispielsweise organische Silyl- oder Stannylgruppen, ferner auch Ylidengruppen, die zusammen mit der Aminogruppe eine Schiff'sche Base bilden. Die genannten organischen Silyl- oder Stannylgruppen sind z.B. die gleichen, die auch mit der 4-Carboxylgruppe am Cephemring eine geschützte Carboxylgruppe zu bilden vermögen.

Die genannten Ylidengruppen sind in erster Linie Arylmethylengruppen worin Aryl insbesondere für einen carbocyclischen, in erster Linie monocyclischen Arylrest, z.B. für gegebenenfalls durch Nitro oder Hydroxy substituiertes Phenyl steht; solche Arylmethylgruppen sind z.B. Benzyliden, 2-Hydroxybenzyliden oder 4-Nitrobenzyliden, ferner gegebenenfalls, z.B. durch Carboxy substituiertes Oxacycloalkyliden, z.B. 3-Carboxy-2-oxacyclohexyliden.

Den Acylrest einer Carbonsäure der Formel (III) einführende Acylierungsmittel sind beispielsweise die Carbonsäure selbst oder reaktionsfähige funktionelle Derivate davon.

Falls eine freie Säure der Formel III, worin alle funktionellen Gruppen ausser der reagierenden Carboxylgruppe geschützt sind, als Acylierungsmittel eingesetzt wird, verwendet man üblicherweise geeignete Kondensationsmittel, wie Carbodiimide, beispielsweise N,N'-Diäthyl-, N,N'-Dipropyl-, N,N'-Diisopropyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazoliniumsalze, beispielsweise N-Aethyl-5-phenyl-isoxazolinium-3'-sulfonat und N-tert.-Butyl-5-methyl-isoxazoliniumperchlorat, oder eine Acylaminoverbindung, z.B. 2-Aethoxy-1-äthoxycarbonyl-1,2-dihydrochinolin.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Ein reaktionsfähiges, d.h. amidbildendes, bzw. esterbildendes, funktionelles Derivat einer Säure der Formel III, worin alle funktionellen Gruppen ausser der reagierenden Säuregruppe geschützt sein können, ist in erster Linie ein Anhydrid einer solchen Säure, inklusive und vorzugsweise ein gemischtes Anhydrid, aber auch ein inneres Anhydrid, d.h. ein entsprechendes Keten. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure oder phosphoriger Säure, oder mit einer schwefelhaltigen Säure, z.B. Schwefelsäure, oder mit Cyanwasserstoffsäure.

Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Weitere reaktionsfähige Säurederivate einer Säure der Formel III sind aktivierte Ester, wie Ester mit vinylogen Alkoholen (d.h. Enolen), wie vinylogen Niederalkenolen, oder Arylester, wie 4-Nitrophenyl- oder 2,4-Dinitrophenylester, heteroaromatische Ester, wie Benztriazol-, z.B. 1-Benztriazolester, oder Diacyliminoester, wie Succinylimino- oder Phthalyliminoester.

Die Acylierung mit einem Säurederivat, wie einem Anhydrid, insbesondere mit einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt. Die reaktionsfähigen Ester, z.B. die 1-Benztriazolester werden auch in Gegenwart eines der genannten Carbodiimide, z.B. N,N-Dicyclohexylcarbodiimid, verwendet.

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, bei Raumtemperatur, wenn notwendig, bei erniedrigter oder erhöhter Temperatur, etwa bei -40° bis etwa 100°, bevorzugt bei -10° bis +40°, und/oder in einer Inertgas-, z.B. Stickstoffatmosphäre.

In einer acylierenden Säure der Formel III oder in einem Säurederivat davon kann eine geschützte Aminogruppe auch in ionischer Form vorliegen, d.h. das Ausgangsmaterial der Formel III kann in Form eines Säureadditionssalzes, vorzugsweise mit einer starken anorganischen Säure, wie einer Halogenwasserstoffsäure, z.B. Salzsäure, oder Schwefelsäure verwendet werden.

Ferner kann ein Säurederivat, wenn erwünscht, in situ gebildet werden. So erhält man z.B. ein gemischtes Anhydrid durch Behandeln einer Säure der Formel III mit entsprechend geschützten funktionellen Gruppen, oder eines geeigneten Salzes davon, wie eines Ammoniumsalzes, z.B. mit einem organischen Amin, wie 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, oder mit einem Halbester eines Kohlensäurehalbhalogenids, z.B. Chlorameisensäureäthylester oder -isobutylester, und verwendet das so erhältliche gemischte Anhydrid ohne Isolierung.

Verfahren b): In einer Ausgangsverbindung der Formel IV ist X Halogen, insbesondere Chlor, aber auch Brom, Jod oder Fluor. Der Thioharnstoff wird in freier Form oder als Salz, insbesondere als

Thiolat eines Alkalimetalls, wie Lithium, Natrium oder Kalium, oder auch als Thiolat einer Ammoniumverbindung, in äquivalenter Menge od in bis zu sechsfachem Ueberschuss, eingesetzt.

Die Reaktion wird im allgemeinen in einem Lösungsmittel, wie Wasser oder einem organischen, nicht-reaktiven Lösungsmittel oder einer Mischung davon, durchgeführt. Als organische Lösungsmittel geeignet sind Alkohole, wie Methanol, Aethanol, Isopropanol, Ketone, wie Aceton, Aether, wie Dioxan oder Tetrahydrofuran, Nitrile, wie Aceto nitril, halogenierte Kohlenwasserstoffe, wie Methylenchlorid, Chlor form oder Tetrachlorkohlenstoff, Ester, wie Aethylacetat, oder Amid wie Dimethylformamid oder Dimethylacetamid, und ähnliche. Die Reakt kann, falls die freien Verbindungen eingesetzt werden, in Gegenwart einer Base durchgeführt werden. Geeignete Basen sind Alkalimetallhydroxide, wie Natrium- oder Kaliumhydroxid, Alkalimetallcarbonate, wie Natrium- oder Kaliumcarbonat, oder organische tertiäre Sticksto basen, wie Triniederalkylamine, z.B. Trimethylamin, Triäthylamin, Aethyl-diisopropylamin, Pyridin und dergleichen. Die Reaktionstemperatur liegt bei Raumtemperatur, oder auch tiefer oder höher, vo zugsweise zwischen -10 bis +100° , insbesondere zwischen 0 bis 40°

Die Reaktion kann auch stufenweise erfolgen, indem zunächst das rin offene Zwischenprodukt mit der Teilformel $Am-C(=NH)-S-CH_2-CO-C(=NOC$ entsteht, das dann in der zweiten Stufe dehydratisiert wird.

<u>Verfahren c):</u> Die Gruppe $R_o$ in einem Ausgangsmaterial der Formel (V) kann freies Hydroxy sein, steht aber vorzugsweise für verestertes Hydroxy oder gegebenenfalls substituiertes Amino. Eine veresterte Hydroxygruppe $R_o$ kann durch eine anorganische oder organische Säure, wie eine starke Mineralsäure, z.B. eine Halogenwasserstoffsäure, wi

Chlorwasserstoff-, Bromwasserstoff- oder Jodwasserstoffsäure, oder eine organische Carbon- oder Sulfonsäure, inklusive Ameisensäure, wie eine entsprechende aliphatische, cycloaliphatische, cycloaliphatischaliphatische, aromatische, araliphatische, heterocyclische oder heterocyclisch-aliphatische Säure, ferner durch ein Kohlensäurehalbderivat verestert sein. So stellt $R_o$ z.B. Halogen, wie Chlor, Brom oder Jod, Niederalkylsulfonyloxy, z.B. Methansulfonyloxy oder Aethansulfonyloxy, Arylsulfonyloxy, z.B. Benzolsulfonyloxy, oder p-Toluolsulfonyloxy, Niederalkanoyloxy, z.B. Acetyloxy oder Propionyloxy, Arylcarbonyloxy, z.B. Benzoyloxy, oder Niederalkoxycarbonyloxy, z.B. Methoxycarbonyloxy oder Aethoxycarbonyloxy, dar. Eine gegebenenfalls substituierte Aminogruppe $R_o$ ist eine primäre, sekundäre oder tertiäre Aminogruppe $-N(R_o^1)(R_o^2)$, worin $R_o^1$ und $R_o^2$ unabhängig voneinander Wasserstoff, oder einen gegebenenfalls substituierten aliphatischen, cycloaliphatischaliphatischen, aromatischen, araliphatischen, heterocyclischen oder heterocyclisch-aliphatischen Rest, oder zusammen eine gegebenenfalls substituierte, gegebenenfalls durch Heteroatome, wie Sauerstoff, Schwefel oder Stickstoff, unterbrochene Polymethylengruppe bedeuten. Solche Aminogruppen $R_o$ sind beispielsweise Amino, Mono- oder Diniederalkylamino, wie Methyl-, Aethyl-, Propyl-, Dimethyl-, Diäthyl-, Dipropyl-, Methyl-äthyl-, oder Methyl-propyl-amino, Mono- oder Dicyclohexylamino, Diphenylamino, Benzyl-, Dibenzyl- oder Phenyläthyl- oder Diphenyläthylamino, Aziridino, Pyrrolidino, Piperidino, Morpholino, Thiomorpholino oder 4-Niederalkylpiperazino.

Die Abspaltung einer Verbindung der Formel $R_o$-H, d.h. von Wasser, einer Säure oder eines Amins, wird vorzugsweise durch Behandeln mit geeigneten Wasser-, Säure- oder Amin-abspaltenden Mittel durchgeführt. Wasser und Amine werden vorzugsweise in Gegenwart eines sauren wasser- bzw. aminabspaltenden Mittels, z.B. einer Säure, vorzugsweise einer starken organischen Carbon- oder Sulfonsäure, wie einer

Halogen-niederalkancarbonsäure, z.B. Trifluoressigsäure, oder einer Arylsulfonsäure, z.B. p-Toluolsulfonsäure, eines geeigneten Säurederivats, wie eines Anhydrids oder insbesondere eines Halogenids, wie Chlorids, in erster Linie einer anorganischen, z.B. Phosphor- oder Schwefel-haltigen, Säure, z.B. Phosphoroxychlorid oder Thionylchlorid wobei man ein solches Derivat, wenn Wasser abgespalten werden soll, üblicherweise in Gegenwart einer Base, wie einer tertiären organische Base, z.B. Pyridin, verwendet, oder eines geeigneten sauren Ionenaustauschers, wie eines Ionenaustauschers auf Sulfonsäure-Basis, z.B. eines sulfonierten Polystyrolionenaustauschers, abgespalten. Zur Abspaltung von Wasser kann man auch dehydratisierende Carbodiimidverbindungen, z.B. Dicyclohexylcarbodiimid, oder dehydratisierende, über Stickstoffatome disubstituierte Carbonylverbindungen, z.B. Carbodiimidazol, verwenden. Dabei verwendet man diese Mittel üblicherweise in Gegenwart eines Lösungsmittels, wie eines gegebenenfalls halogenierten aliphatischen, cycloaliphatischen oder aromatischen Kohlenwasserstoffs, z.B. Benzol oder Toluol, oder eines Lösungsmittelgemisches, wobei man geeignete saure Mittel, wie Trifluoressigsäure gleichzeitig auch als Lösungsmittel verwenden kann. Wenn notwendig, verwendet man zusätzlich ein wasser-absorbierendes Mittel oder einen Wasserabscheider und arbeitet unter Kühlen oder Erwärmen und/oder in einer Inertgas-, z.B. Stickstoffatmosphäre.

Vorzugsweise bedeutet $R_o$ in einem Ausgangsmaterial der Formel V eine veresterte Hydroxygruppe und man spaltet erfindungsgemäss eine Säure der Formel $H-R_o$ ab. Ueblicherweise verwendet man zu diesem Zweck basische Säure-abspaltende und/oder -neutralisierende Mittel, wie z.B. anorganische Base, wie verdünnte Alkalimetallhydroxide, z.B. Natrium- oder Kaliumhydroxid, wobei man neben Wasser auch organische Lösungsmittel, wie geeignete Ketone, z.B. Aceton, oder Aether, z.B. Dioxan, oder wässrige Gemische davon verwenden und bei einem pH-Wert von höchstens etwa 9, wenn notwendig, unter Kühlen oder Erwärmen und/oder in einer Inertgas-, z.B. Stickstoffatmosphäre arbeiten kann.

Vorzugsweise verwendet man als Säure-abspaltende Mittel tert.-Amine, insbesondere gute Protonenakzeptoren, die den Lactamring nicht angreifen, in erster Line tert.-aliphatische oder tert.-cycloaliphatische Mono- und Diamine, wie Triniederalkylamine, z.B. Trimethylamin, Triäthylamin oder Aethyl-diisopropylamin, oder bicyclische Diazaverbindungen mit einer Amidin-artigen Anordnung der Ringstickstoffatome, z.B. 1,5-Diazabicyclo [4,3,0]non-5-en oder 1,5-Diazabicyclo[5,4,0]undec-5-en. Ferner kann man basische Ionenaustauscher, z.B. auf Ammoniumhydroxid-Basis, ebenfalls als Säure-abspaltende Mittel einsetzen. Gewisse veresterte Hydroxygruppen $R_o$, insbesondere Sulfonyloxy, z.B. Methylsulfonyloxygruppen lassen sich in Form einer Säure der Formel $R_o$-H auch durch Absorption, z.B. an Silikagel, Aluminiumoxyd, etc., und Elution (Chromatographie), aus Verbindungen der Formel V abspalten.

Die oben beschriebenen Säureabspaltungen werden in Abwesenheit, üblicherweise aber in Gegenwart eines Lösungsmittels, wie eines gegebenenfalls halogenierten Kohlenwasserstoffs aliphatischen, cycloaliphatischen oder aromatischen Charakters, wie Methylenchlorid, eines Niederalkanons, z.B. Aceton, oder Aethers, z.B. Tetrahydrofuran oder Dioxan, oder eines Lösungsmittelgemisches, inkl. eines wässrigen Gemisches, wenn notwendig unter Kühlen oder Erwärmen, bei -30° bis 150°, bevorzugt bei etwa -10° bis 60°, und/oder in einer Inertgas-, z.B. Stickstoffatmosphäre vorgenommen.

Verfahren d): Die Methylierung der Hydroxyiminomethylengruppe in einer Verbindung der Formel VI erfolgt auf an sich bekannte Weise durch Behandeln mit einem Methylierungsmittel. Im Ausgangsmaterial sind ausser der Hydroxyiminogruppe alle gegebenenfalls vorhandenen weiteren funktionellen Gruppen vorzugsweise geschützt.

Geeignete Methylierungsmittel sind beispielsweise Diazomethan, reaktionsfähige Ester von Methanol, wie Methylhalogenide, z.B. Methyliodid, Sulfonsäureester, z.B. Methansulfonsäure-, Trifluormethansulfonsäure- oder p-Toluolsulfonsäuremethylester, oder Schwefelsäureester, z.B. Dimethylsulfat, Dimethylacetale, z.B. 2,2-Dimethoxypropan, Orthoester, z.B. Orthoameisensäuretrimethylester, Trimethyloxoniumsalze, z.B. Trimethyloxoniumfluorantimonat, -hexachlorantimonat, -hexafluorphosphat oder -tetrafluorborat, Dimethoxycarboniumsalze, z.B. Dimethoxycarbonium-hexafluorphosphat, oder Dimethylhaloniumsalze, z.B. Dimethylbromonium-hexafluorantimonat, oder 3-Aryl-1-methyl-triazenverbindungen, z.B. 3-p-Tolyl-1-methyltriazen. Die Methylierung wird auf an sich bekannte Weise, üblicherweise in einem inerten Lösungsmittel, wie einem Aether, z.B. Dioxan oder Tetrahydrofuran, oder einem gegebenenfalls halogenierten Kohlenwasserstoff, wie Benzol, Chlorbenzoyl, Methylenchlorid, oder dergleichen, gegebenenfalls in Gegenwart geeigneter Kondensationsmittel, wie Basen oder Säuren, unter Kühlen oder Erwärmen, z.B. bei Temperaturen zwischen etwa -20° bis etwa 100°, durchgeführt.

Verfahren e): Eine reaktionsfähige veresterte Hydroxygruppe X in einer Verbindung der Formel VIII ist eine durch eine starke anorganische oder organische Säure veresterte Hydroxygruppe. Entsprechende Gruppen X sind insbesondere Halogen, z.B. Chlor, Brom oder bevorzugt Jod, ferner Sulfonyloxygruppen, wie Niederalkan- oder Arensulfonyloxygruppen, z.B. Methan-, Aethan-, Benzol- oder Toluolsulfonyloxygruppen, oder Halogesulfongruppen, z.B. die Chlorsulfongruppe, und dergleichen.

Die Veresterung der gegebenenfalls in Salzform vorliegenden 4-Carboxylgruppe wird, auf an sich bekannte Weise, in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, eines N,N-Diniederalkyl-anilins, z.B. N,N-Dimethylanilin, eines cyclischen tertiären Amins, wie eines N-niederalkylierten Morpholins, z.B. N-Methylmorpholin, einer Base vom Pyridin-Typ, z.B. Pyridin, einer

anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder einer quaternären Ammoniumbase, wie eines Tetraalkylammoniumhydroxids, -carbonats oder -Hydrogencarbonats, z.B. worin Alkyl, Methyl, Aethyl, Propyl, Isopropyl, Butyl oder dergleichen ist, oder eines Oxirans, beispielsweise eines niederen 1,2-Alkylenoxids, wie Aethylenoxid oder Propylenoxid, durchgeführt.

Bevorzugt wird die Carbonsäure der Formel VII zunächst in ein Salz einer der genannten organischen oder anorganischen Basen, insbesondere in das Natriumsalz, übergeführt und dieses mit einer Verbindung der Formel VIII umgesetzt.

Eine Verbindung der Formel VIII kann auch in situ hergestellt werden. Beispielsweise kann man eine Verbindung der Formel VIII worin X Chlor bedeutet durch Behandeln mit Natriumjodid in einem Lösungsmittel, z.B. in Aceton oder Acetonitril, in eine Verbindung der Formel VIII überführen worin X Jod bedeutet, oder man kann die Veresterung mit einer Chlorverbindung der Formel VIII in Gegenwart von Natriumjodid durchführen.

Die Veresterungsreaktion wird in einem geeigneten Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, bei Raumtemperatur, wenn notwendig, bei erniedrigter oder erhöhter Temperatur, etwa bei -40° bis etwa 100°, bevorzugt bei -10° bis +40°, und/oder in einer Inertgas-, z.B. Stickstoffatmosphäre.

Verfahren f): Eine geschützte Aminogruppe Am setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Brom-niederalkoxycarbonylaminogruppe in eine 2-Jod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 2-Acyl-niederalk-1-en-1-yl-amino, z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silyl- oder Stannylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure,

in die freie Aminogruppe übergeführt werden. Eine Phosphor-, Phosphon- oder Phosphin-amidogruppe kann z.B. durch Behandeln mit einer phosphorhaltigen Säure, wie einer Phosphor-, Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, in die freie Aminogruppe übergeführt werden.

Die beschriebenen Spaltungsreaktionen werden im übrigen unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Bevorzugt werden selektive Abspaltungsreaktionen durchgeführt, d.h. solche bei denen die Estergruppierung und die Methoxyiminogruppe entweder gar nicht oder nur wenig angegriffen werden. Eine solche bevorzugte Methode ist die acidolytische Abspaltung einer tert.-Niederalkoxycarbonylgruppe, insbesondere der tert.-Butoxycarbonylgruppe, mittels einer geeigneten Säure, insbesondere Trifluoressigsäure, gegebenenfalls in einem inerten Lösungsmittel, wie einem gegebenenfalls halogenierten Kohlenwasserstoff, z.B. Methylenchlorid, bei Temperaturen von etwa -20° bis etwa 50°, bevorzugt bei 0° bis Raumtemperatur.

Säureadditionssalze von Verbindungen der Formel I, worin Am eine freie Aminogruppe bedeutet, erhält man in üblicher Weise, z.B. durch Behandeln mit einer Säure oder einem geeigneten Anionenaustauschreagens.

Die Verfahren umfassen auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder in situ, gegebenenfalls unter den Reaktionsbedingungen, gebildet werden.

Die Ausgangsverbindungen der Formeln II bis VIII sind bekannt oder können analog bekannten Verfahren hergestellt werden.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusamme oder im Gemisch mit anorganischen oder organischen, festen oder flüssigen, pharmazeutisch verwendbaren Trägerstoffen enthalten, die sich zur enteralen Verabreichung eignen. Zur enteralen Verabreichung verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Suppositorien sind in erster Linie Fettemulsionen ode -suspensionen.

Die vorliegenden pharmazeutischen Präparate, die, wenn erwünscht, weitere pharmakologisch wertvolle Stoffe enthalten können, werden in an sich bekannter Weise, z.B. mittels konventioneller Mischungs-, Lösungs-, oder Lyophilisierungsverfahren, hergestellt und enthalten von etwa 0,1% bis 100%, insbesondere von etwa 1% bis etwa 50%, Lyophilisate bis zu 100% des Aktivstoffes. Je nach Art der Infektion, Zustand des infizierten Organismus verwendet man tägliche Dosen von etwa 0,25 g bis etwa 2 g p.o. zur Behandlung von Warmblütern von etwa 70 kg Gewicht.

Die folgenden Beispiele dienen zur Illustration der Erfindung; vor- und nachstehend werden Temperaturen in Celsiusgraden angegeben. Die Methoxyiminogruppe hat die syn-Konfiguration.

Beispiel 1: Eine eisgekühlte Lösung von 10 g 7β-[2-(2-tert.Butoxycarbonylamino-4-thiazolyl)-2-methoxyiminoacetamido]-3-cephem-4-carbonsäure-natriumsalz in 40 ml Dimethylformamid wird mit 14 g α-Joddiäthylcarbonat versetzt und zunächst 15 Minuten unter Eiskühlung und dann 45 Minuten bei Raumtemperatur gerührt. Die Reaktionsmischung wird auf Eiswasser gegossen und mit Aethylacetat ausgeschüttelt. Die organische Phase wird nacheinander mit wässriger Natriumhydrogencarbonatlösung, Wasser und gesättigter, wässriger Natriumchloridlösung gewaschen, im Vakuum eingeengt und am Hochvakuum getrocknet. Das Rohprodukt wird an 200 g Kieselgel mit Toluol und Toluol enthaltend steigende Mengen Aethylacetat (10 bis 30%) chromatographiert. Die das Produkt vom Rf-Wert ~ 0,26 (Silikagel; Aethylacetat) enthaltenden Fraktionen werden vereinigt und im Vakuum eingedampft. Nach dispergieren des Rückstandes mit einer Mischung von Diäthyläther und Hexan und trocknen im Hochvakuum erhält man den 7β-[2-(2-tert.-Butoxycarbonylamino-4-thiazolyl)-2-methoxyiminoacetamido]-3-cephem-4-carbonsäure-1-äthoxycarbonyloxyäthylester.
IR-Spektrum ($CH_2Cl_2$): charakteristische Absorptionsbanden bei 3400, 1796, 1765, 1728, 1690; 1548 $cm^{-1}$; UV-Spektrum (Aethanol): $\lambda_{max}$ = 228 ($\varepsilon$ =18 800), 260 ($\varepsilon$ = 17000) mμ.

Beispiel 2: Eine Lösung von 6 g 7β-[2-(2-tert.Butoxycarbonylamino-4-thiazolyl)-2-methoxyiminoacetamido]-3-cephem-4-carbonsäure-1-äthoxycarbonyloxyäthylester in 30,5 ml Methylenchlorid wird mit 30,5 ml Trifluoressigsäure versetzt und eine Stunde bei Raumtemperatur gerührt. Das Reaktionsgemisch wird zweimal mit einer Mischung von Toluol und Chloroform 1:1 und einmal mit Diäthyläther versetzt und jedesmal im Vakuum abgedampft. Der Rückstand wird in Aethylacetat aufgenommen. Die Lösung wird dreimal mit konzentrierter wässriger Natriumhydrogencarbonatlösung und einmal mit gesättigter, wässriger Natriumchloridlösung gewaschen und im Vakuum eingedampft. Der Rückstand wird an Kieselgel mit Toluol und Toluol enthaltend steigende Mengen Aethylacetat (10 bis 50%) chromatographiert. Die das reine Produkt vom Rf-Wert ~ 0.22 (Silikagel; Aethylacetat) enthaltenden Fraktionen werden vereinigt und im Vakuum eingedampft. Der Rückstand wird mit Diäthyläther digeriert, im Hochvakuum getrocknet und ergibt den 7β-[2-(2-Amino-4-thiazolyl)-2-methoxyiminoacetamido]-3-cephem-4-carbonsäure-1-äthoxycarbonyloxyäthylester. Zersetzungspunkt: 122-128° ; IR-Spektrum ($CH_2Cl_2$): charakteristische Absorptionsbanden bei 3400, 1785 (breit), 1765, 1685, 1608, 1530 $cm^{-1}$; UV-Spektrum (Aethanol): $\lambda_{max}$ = 235 ($\varepsilon$ = 16500) und Schulter bei 258 mμ.

Beispiel 3: Analog den vorstehenden Beispielen wird ausgehend vom 7β-[2-(2-tert.Butoxycarbonylamino-4-thiazolyl)-2-methoxyiminoacetamido]-3-cephem-4-carbonsäure-natriumsalz durch Umsetzung mit Jodmethyl-äthylcarbonat der 7β-[2-(2-tert.Butoxycarbonylamino-4-thiazolyl)-2-methoxyiminoacetamido]-3-cephem-4-carbonsäure-äthoxycarbonyloxymethylester hergestellt, der nach Behandlung mit Trifluoressigsäure den 7β-[2-(2-Amino-4-thiazolyl)-2-methoxyiminoacetamido]-3-cephem-4-carbonsäure-äthoxycarbonylaminomethylester ergibt. IR-Spektrum ($CH_2Cl_2$): charakteristische Absorptionsbanden bei u.a. 3400; 1782; 1760; 1610; 1535 $cm^{-1}$ .

Beispiel 4: Zu einer auf 0° gekühlten Lösung von 5 g 7β-[2-(2-Amino-4-thiazolyl)-2-methoxyiminoacetamido]-3-cephem-4-carbonsäure-1-äthoxycarbonyloxyäthylester (freie Base) in 50 ml $CH_2Cl_2$ werden 61 ml einer 0.18M HCl-Lösung in $CH_2Cl_2$ (1.1 Moläquivalent, hergestellt durch Einleiten von trockenem gasförmigem Chlorwasserstoff in trockenes $CH_2Cl_2$) gegeben. Nach 10-minütigem Rühren wird die Lösung mit Diäthyläther versetzt, wobei ein Niederschlag ausfällt. Das Gemisch wird 1/2 Stunde bei 0° nachgeführt, der Niederschlag abfiltriert, mit Diäthyläther gewaschen und im Hochvakuum bei 30° getrocknet. Das erhaltene rohe

Hydrochlorid, wird in ca. 50 ml $CH_2Cl_2$ gelöst, etwas eingeengt und über Nacht bei etwa +5° stehen gelassen. Das ausgefallene 7β-[2-(2-Amino-4-thiazolyl)-2-methoxyiminoacetamido]-3-cephem-4-carbonsäure-1-äthoxycarbonyloxyäthylester-hydrochlorid wird abfiltriert, mit wenig $CH_2Cl_2$ und Diäthyläther gewaschen und wie zuvor getrocknet.

Beispiel 5: Analog Beispiel 4 wird durch Umsatz von 5 g 7β-[2-(2-Amino-4-thiazolyl)-2-methoxyiminoacetamido]-3-cephem-4-carbonsäure-1-äthoxycarbonyloxyäthylester (freie Base) in Methylenchlorid mit 27.2 ml einer 0.46M Bromwasserstofflösung in Methylenchlorid das 7β-[2-(2-Amino-4-thiazolyl)-2-methoxyiminoacetamido]-3-cephem-4-carbonsäure-1-äthoxycarbonyloxyäthylesterhydrobromid erhalten.

Beispiel 6: Eine Suspension von 0.405 g 7β-[2-(2-Amino-4-thiazolyl)-2-methoxyiminoacetamido]-3-cephem-4-carbonsäure-natriumsalz in 4 ml Dimethylformamid wird mit 0,6 g Joddiäthylcarbonat versetzt und 40 Minuten bei ca. -5° gerührt. Die erhaltene Reaktionsmischung wird in eisgekühlten Phosphatpuffer pH 8 gegossen, 15 Minuten gerührt und mit Aethylacetat extrahiert. Die organische Phase wird nacheinander mit Phosphatpuffer und gesättigter wässriger Natriumchloridlösung ausgeschüttelt und im Vakuum auf ein Volumen von ca. 15 ml eingeengt. Die erhaltene Lösung wird mit 8 ml einer 1.46n Salzsäurelösung in Methylenchlorid und anschliessend mit Diäthyläther versetzt. Der erhaltene Niederschlag wird zweimal aus Methylenchlorid/Diäthyläther umgefällt und ergibt nach Trocknen das 7β-[2-(2-Amino-4-thiazolyl)-2-methoxyiminoacetamido]-3-cephem-4-carbonsäure-1-äthoxycarbonyloxyäthylester-hydrochlorid; Schmelzpunkt ab 145° (Zersetzung). Rf~ 0.20 (Silikagel; Aethylacetat); IR-Spektrum (Nujol): charakteristische Absorptionsbanden bei 3400, 1783 (b), 1766, 1740 (sh), 1680, 1605 $cm^{-1}$.

Beispiel 7: Man lässt eine Mischung von 0,18 ml Dimethylformamid, 0,2 ml Oxalylchlorid und 8 ml Methylenchlorid während 30 Minuten bei ca. -5° reagieren, versetzt sie anschliessend mit 0,57 g 2-(2-tert.-Butoxycarbonylamino-4-thiazolyl)-2-methoxyiminoessigsäure und 0.3 ml N-Methylmorpholin und lässt weitere 30 Minuten bei -5° rühren. Nach Zugabe einer Lösung von 0.71 g 7β-Amino-3-cephem-4-carbonsäure-1-äthoxycarbonyloxyäthylester-hydrochlorid und 0.6 ml N-Methylmorpholin in 7 ml Methylenchlorid wird die Reaktionsmischung 30 Minuten bei -5° und 30 Minuten bei 0° weitergerührt, mit Aethylacetat verdünnt und nacheinander mit 1n Salzsäure, gesättigter wässriger Natriumhydrogencarbonatlösung und gesättigter wässriger Natriumchloridlösung gewaschen. Die organische Phase wird über Natriumsulfat getrocknet und im Vakuum eingedampft. Der Rückstand wird mit Toluol enthaltend 10-20% Aethylacetat an Silikagel chromatographiert und ergibt den 7β-[2-(2-Amino-4-thiazolyl)-2-Z-methoxyiminoacetamido]-3-cephem-4-carbonsäure-1-äthoxycarbonyloxyäthylester mit den gleichen Eigenschaften wie im Beispiel 2 angegeben.

Das Ausgangsmaterial wird wie folgt hergestellt:
Eine Suspension von 2 g 7β-Amino-3-cephem-4-carbonsäure in 20 ml Dimethylacetamid wird mit 1.5 ml 1,5-Diazabicyclo[5.4.0]undec-5-en 15 Minuten bei Raumtemperatur gerührt, auf -10° gekühlt, mit 2.6 g α-Joddiäthylcarbonat versetzt und 30 Minuten bei -10° reagieren gelassen. Das Reaktionsgemisch wird auf Eiswasser gegossen, der pH-Wert auf 6 eingestellt,und mit Aethylacetat extrahiert. Die organische Phas wird mit gesättigter wässriger Natriumchloridlösung ausgeschüttelt, über Natriumsulfat getrocknet und im Vakuum auf ca. 30 ml eingedampft. Die erhaltene Lösung wird mit 1.3 Moläquivalenten einer äthanolischen Salzsäurelösung versetzt und nach 30 Minuten Rühren unter Eiskühlung mit Diäthyläther behandelt. Der ausfallende Niederschlag von 7β-Amino-3-cephem-4-carbonsäure-1-äthoxycarbonyloxyäthylester-hydrochlorid wird abfiltriert, mehrmals mit Diäthyläther gewaschen und getrocknet. Schmelzpunkt ca. 200° (Zersetzung); Rf ~ 0.23 (Silikagel, Toluol:Essigester 1:1).

Beispiel 8: Kapseln, enthaltend 0,250 g 7β-[2-(2-Amino-4-thiazolyl)-2-methoxyiminoacetamido]-3-cephem-4-carbonsäure-1-äthoxycarbonyloxyäthylester werden wie folgt hergestellt:

Zusammensetzung (für 100'000 Kapseln):

| | |
|---|---|
| 7β-[2-(2-Amino-4-thiazolyl)-2-methoxy-imino-acetamido]-3-cephem-4-carbonsäure-1-äthoxy-carbonyloxyäthylester | 25'000 g |
| Weizenstärke | 2'500 g |
| Magnesiumstearat | 1'000 g |

Der 7β-[2-(2-Amino-4-thiazolyl)-2-methoxyiminoacetamido]-3-cephem-4-carbonsäure-1-äthoxycarbonyloxyäthylester, die Weizenstärke und das Magnesiumstearat werden gut miteinander vermischt und in Kapseln Nr. 1 abgefüllt.

Beispiel 9: Kapseln enthaltend 0,5 g 7β-[2-(2-Amino-4-thiazolyl)-2-methoxyiminoacetamido]-3-cephem-4-carbonsäure-1-äthoxycarbonyloxy-1-äthylester werden wie folgt hergestellt:

Zusammensetzung (für 2000 Kapseln):

| | |
|---|---|
| 7β-[2-(2-Amino-4-thiazolyl)-2-methoxyimino-acetamido]-3-cephem-4-carbonsäure-1-äthoxy-carbonyloxyäthylester | 1000'00 g |
| Polyvinylpyrrolidon | 15'00 g |
| Maisstärke | 115'00 g |
| Magnesiumstearat | 20'00 g |

Man befeuchtet den 7β-[2-(2-Amino-4-thiazolyl)-2-methoxyiminoacetamido]-3-cephem-4-carbonsäure-1-äthoxycarbonyloxyäthylester mit 300 ml einer Lösung des Polyvinylpyrrolidons in 95%-igem Aethanol, treibt das Gemisch durch ein Sieb mit 3 mm Maschenweite und trocknet das Granulat unter vermindertem Druck bei 40-50°. Man siebt durch ein Sieb mit 0,8 mm Maschenweite, gibt die Maisstärke und das Magnesiumstearat zu, vermischt und füllt das Gemisch in Steckkapseln (Grösse 0) ab.

Beispiel 10: Tabletten, enthaltend 250 mg 7β-[2-(2-Amino-4-thiazolyl)-2-methoxyiminoacetamido]-3-cephem-4-carbonsäure-1-äthoxycarbonyloxyäthylester werden wie folgt hergestellt:

Zusammensetzung (für 1 Tablette):

| | |
|---|---|
| 7β-[2-(2-Amino-4-thiazolyl)-2-methoxyimino-acetamido]-3-cephem-4-carbonsäure-1-äthoxycarbonyl-oxyäthylester | 250 mg |
| Mikrokristalline Cellulose | 80 mg |
| Natrium-carboxymethyl-stärke | 10 mg |
| Magnesiumstearat | 3 mg |
| Talk | 7 mg |
| | 350 mg |

Der Wirkstoff wird mit den Zusatzstoffen homogen vermischt und zu Tabletten gepresst.

Zur Herstellung von Filmdragées werden die Tabletten je mit 1 mg wässrigem Lack überzogen.

Anstelle von Natriumcarboxymethyl-stärke kann auch Natrium-carboxymethylcellulose eingesetzt werden.

Anstelle der freien Base kann in den Beispielen 6 bis 8 auch ein Salz, z.B. das Hydrochlorid oder Hydrobromid, als Wirksubstanz eingesetzt werden.

Patentansprüche (für alle benannten Länder ausser Oesterreich)

1. Verbindungen der Formel

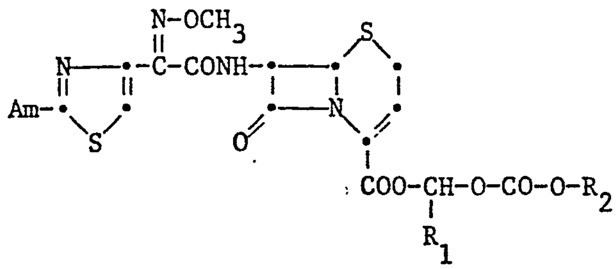

(I) ,

worin $R_1$ Wasserstoff oder Niederalkyl, $R_2$ Niederalkyl und Am eine gegebenenfalls geschützte Aminogruppe bedeuten, und Säureadditionssalze von solchen Verbindungen, worin Am eine freie Aminogruppe bedeutet.

2. Verbindungen gemäss Anspruch 1, worin Am eine freie Aminogruppe bedeutet.

3. Verbindungen gemäss Anspruch 1 oder 2, worin Am eine freie Aminogruppe, $R_1$ Wasserstoff oder Methyl und $R_2$ Aethyl bedeutet.

4. Die Verbindung gemäss Anspruch 1, worin Am eine freie Aminogruppe, $R_1$ Methyl und $R_2$ Aethyl bedeuten.

5. Die Säureadditionssalze einer Verbindung gemäss einem der Ansprüche 1-4.

6. Das Hydrochlorid einer Verbindung gemäss einem der Ansprüche 1-4.

7. Das Hydrobromid einer Verbindung gemäss einem der Ansprüche 1-4.

8. Pharmazeutische Präparate enthaltend eine der in den Ansprüchen 1-7 beschriebenen Verbindungen.

9. Verbindungen gemäss einem der Ansprüche 1-7 zur Verwendung als orale Antibiotika.

10. Verwendung von Verbindungen der Formel I und ihrer pharmazeutisc annehmbaren Salze bei der Bekämpfung von bakteriellen Infektionen.

11. Verfahren zur Herstellung von Verbindungen der Formel I und der Säureadditionssalze von solchen Verbindungen, worin Am eine freie Aminogruppe bedeutet, gemäss Anspruch 1, dadurch gekennzeichnet, dass man

a) die 7β-Aminogruppe in einer Verbindung der Formel

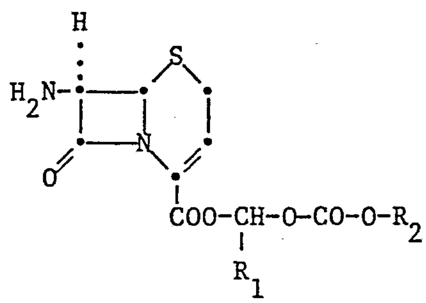

(II) ,

worin die Aminogruppe gegebenenfalls durch eine die Acylierung erlaubende Gruppe substituiert ist und worin $R_1$ und $R_2$ die unter Formel I gegebenen Bedeutungen haben, durch Behandeln mit einem den Acylrest einer Carbonsäure der Formel

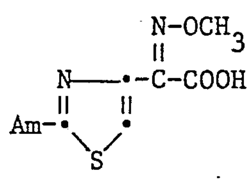

(III)

einführenden Acylierungsmittel, worin Am die unter Formel I angegebenen Bedeutungen hat, acyliert, oder

b) eine Verbindung der Formel

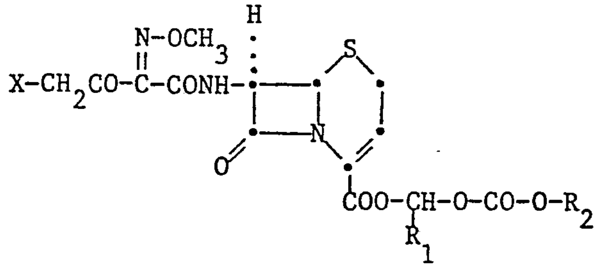

(IV) ,

worin X Halogen bedeutet, $R_1$ und $R_2$ die unter Formel I genannten Bedeutungen haben, mit einem Thioharnstoff der Formel Am-CS-$NH_2$ oder einem Salz davon kondensiert, oder

c) aus einer Verbindung der Formel

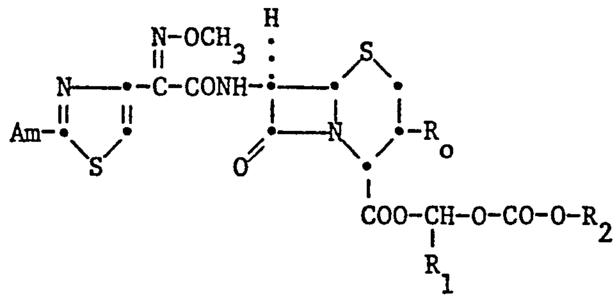

(V) ,

worin Am, $R_1$ und $R_2$ die unter Formel I angegebenen Bedeutungen haben und $R_o$ Hydroxy, verestertes Hydroxy oder gegebenenfalls substituiertes Amino ist, eine Gruppe H-$R_o$ abspaltet, oder

d) zur Herstellung einer Verbindung der Formel I, worin Am, $R_1$ und $R_2$ die unter Formel I gegebenen Bedeutungen haben, in einer Verbindung der Formel

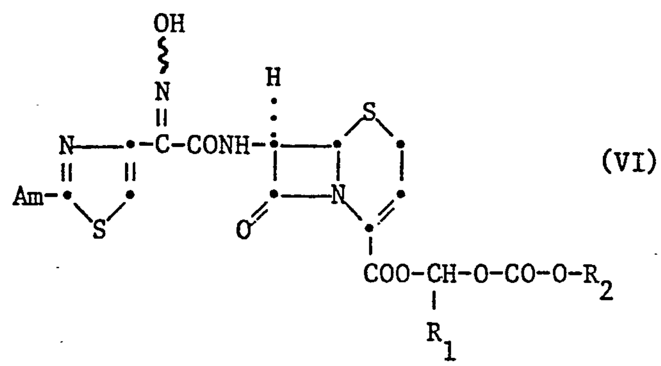

(VI) ,

worin Am, $R_1$ und $R_2$ die unter Formel I gegebenen Bedeutungen haben, die Hydroxyiminogruppe methyliert, oder

e) zur Herstellung einer Verbindung der Formel I, worin Am, $R_1$ und $R_2$ die unter Formel I genannten Bedeutungen haben, in einer Verbindung der Formel

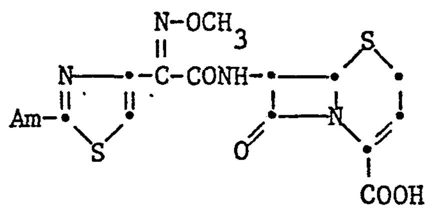

(VII) ,

worin Am die unter Formel I gegebene Bedeutung hat, die gegebenenfall in Salzform vorliegende Carboxylgruppe in 4-Stellung des Cephemringes durch Behandlung mit einem Veresterungsmittel der Formel

$$X-\underset{R_1}{\underset{|}{CH}}-O-CO-O-R_2 \qquad \text{(VIII)} ,$$

worin X eine reaktionsfähige, veresterte Hydroxygruppe ist und $R_1$ und $R_2$ die unter Formel I angegebenen Bedeutungen haben, in eine verester te Carboxylgruppe überführt, und/oder

f) zur Herstellung einer Verbindung der Formel I, worin Am eine frei Aminogruppe ist und $R_1$ und $R_2$ die unter Formel I gegebenen Bedeutung haben, aus einer Verbindung der Formel I, worin Am eine geschützte Aminogruppe bedeutet, und $R_1$ und $R_2$ die unter Formel I gegebenen Bedeutungen haben, die Aminoschutzgruppe abspaltet und durch Wasserstoff ersetzt, und, wenn erwünscht, eine erhaltene Verbindung worin Am eine freie Aminogruppe ist in ein Salz oder ein erhaltenes Salz in eine freie Verbindung oder in ein anderes Salz überführt.

Patentansprüche (für Oesterreich)

1. Verfahren zur Herstellung von Verbindungen der Formel

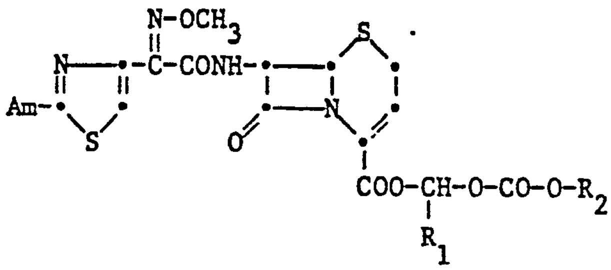

(I) ,

worin $R_1$ Wasserstoff oder Niederalkyl, $R_2$ Niederalkyl und Am eine gegebenenfalls geschützte Aminogruppe bedeuten, und Säureadditionssalzen von solchen Verbindungen, worin Am eine freie Aminogruppe bedeutet, dadurch gekennzeichnet, dass man

a) die 7β-Aminogruppe in einer Verbindung der Formel

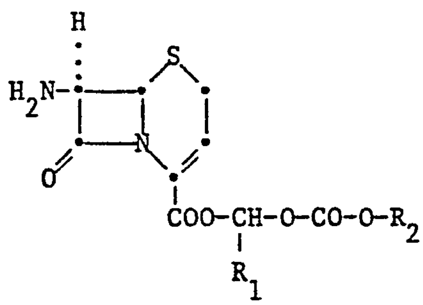

(II) ,

worin die Aminogruppe gegebenenfalls durch eine die Acylierung erlaubende Gruppe substituiert ist und worin $R_1$ und $R_2$ die unter Formel I gegebenen Bedeutungen haben, durch Behandeln mit einem den Acylrest einer Carbonsäure der Formel

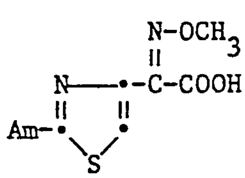

(III)

einführenden Acylierungsmittel, worin Am die unter Formel I angegebenen Bedeutungen hat, acyliert, oder

b) eine Verbindung der Formel

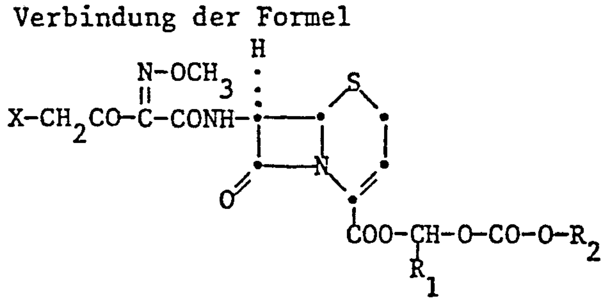

(IV) ,

worin X Halogen bedeutet, $R_1$ und $R_2$ die unter Formel I genannten Bedeutungen haben, mit einem Thioharnstoff der Formel $Am-CS-NH_2$ oder einem Salz davon kondensiert, oder

c) aus einer Verbindung der Formel

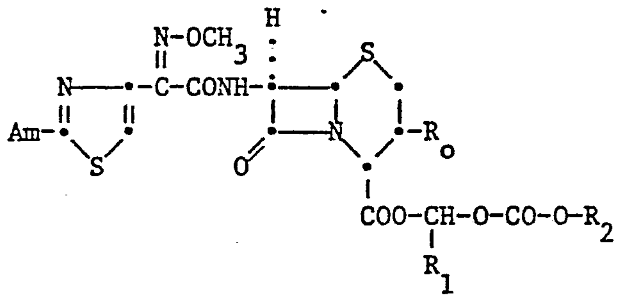

(V) ,

worin Am, $R_1$ und $R_2$ die unter Formel I angegebenen Bedeutungen haben und $R_o$ Hydroxy, verestertes Hydroxy oder gegebenenfalls substituiert Amino ist, eine Gruppe $H-R_o$ abspaltet, oder

d) zur Herstellung einer Verbindung der Formel I, worin Am, $R_1$ und die unter Formel I gegebenen Bedeutungen haben, in einer Verbindung der Formel

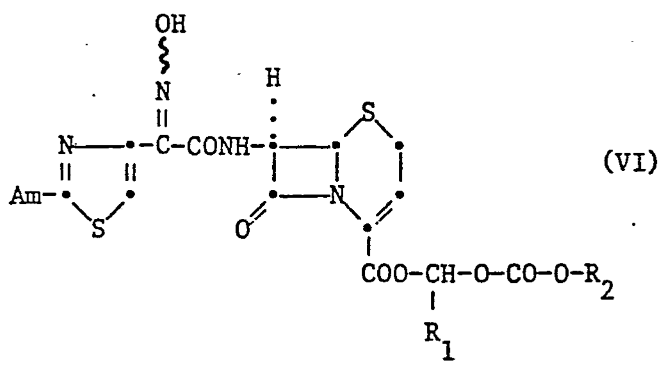

(VI) ,

worin Am, $R_1$ und $R_2$ die unter Formel I gegebenen Bedeutungen haben, die Hydroxyiminogruppe methyliert, oder

e) zur Herstellung einer Verbindung der Formel I, worin Am, $R_1$ und $R_2$ die unter Formel I genannten Bedeutungen haben, in einer Verbindung der Formel

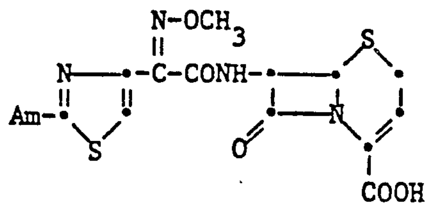

(VII) ,

worin Am die unter Formel I gegebene Bedeutung hat, die gegebenenfall in Salzform vorliegende Carboxylgruppe in 4-Stellung des Cephemringes durch Behandlung mit einem Veresterungsmittel der Formel

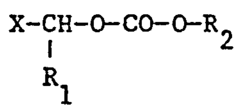

(VIII) ,

worin X eine reaktionsfähige, veresterte Hydroxygruppe ist und $R_1$ und $R_2$ die unter Formel I angegebenen Bedeutungen haben, in eine veresterte Carboxylgruppe überführt, und/oder

f) zur Herstellung einer Verbindung der Formel I, worin Am eine freie Aminogruppe ist und $R_1$ und $R_2$ die unter Formel I gegebenen Bedeutungen haben, aus einer Verbindung der Formel I, worin Am eine geschützte Aminogruppe bedeutet, und $R_1$ und $R_2$ die unter Formel I gegebenen Bedeutungen haben, die Aminoschutzgruppe abspaltet und durch Wasserstoff ersetzt, und, wenn erwünscht, eine erhaltene Verbindung worin Am eine freie Aminogruppe ist in ein Salz oder ein erhaltenes Salz in eine freie Verbindung oder in ein anderes Salz überführt.

2. Verfahren zur Herstellung von Verbindungen der Formel I gemäss Anspruch 1, worin Am eine freie Aminogruppe bedeutet.

3. Verfahren zur Herstellung von Verbindungen der Formel I gemäss Anspruch 1 oder 2, worin Am eine freie Aminogruppe, $R_1$ Wasserstoff oder Methyl und $R_2$ Aethyl bedeutet.

4. Verfahren zur Herstellung von Verbindungen der Formel I gemäss Anspruch 1, worin Am eine freie Aminogruppe, $R_1$ Methyl und $R_2$ Aethyl bedeuten.

5. Verfahren zur Herstellung von Säureadditionssalzen einer Verbindung der Formel I gemäss einem der Ansprüche 1-4.

6. Verfahren zur Herstellung des Hydrochlorids einer Verbindung der Formel I gemäss einem der Ansprüche 1-5.

7. Verfahren zur Herstellung des Hydrobromids einer Verbindung der Formel I gemäss einem der Ansprüche 1-5.